# FASCICULE DE BREVET EUROPEEN

(11) **EP 1 424 061 B1**
(45) Date de publication et mention de la délivrance du brevet: **02.09.2009**
(21) Numéro de dépôt: 03292930.9
(22) Date de dépôt: 26.11.2003
(51) Int. Cl.: A61K 8/84, A61Q 5/00

(54) **Procédé de traitement cosmétique capillaire conférant aux cheveux des propriétés cosmétiques durables**
Kosmetisches Haarbehandlungsverfahren um dem Haar kosmetische Eigenschaften zu verleihen
Cosmetic hair treatment process for imparting enduring cosmetic properties to hair

(30) Priorité: 29.11.2002 FR 0215076
(43) Date de publication de la demande: 02.06.2004
(73) Titulaire: L'ORÉAL, 75008 Paris (FR)
(72) Inventeur: Vic, Gabin, 60280 Venette (FR); Livoreil, Aude, 75006 Paris (FR); Daubresse, Nicolas, 78170 La Celle St Cloud (FR)
(74) Mandataire: Casalonga, Axel

(56) Documents cités:
- US-A- 5 523 080
- US-A1- 2002 012 639
- US-A1- 2002 041 855
- US-A1- 2002 108 188

## Description

La présente invention est relative à un procédé de traitement cosmétique capillaire conférant aux cheveux des propriétés cosmétiques durables, et en particulier rémanentes aux shampooings.

Il est connu de traiter les cheveux par adsorption physicochimique ou fixation de matière sur la surface des cheveux afin d'augmenter leur brillance, de les colorer ou de les rendre plus résistants à la chaleur à la lumière, etc.

Le document FR 2607002 décrit le greffage sur le cheveu préalablement réduit d'un polymère polyhaloacétylé.

Les brevets US 4,973,475, US 5,087,733 et US 5,211,942 décrivent le greffage de composés thiolés sur des cheveux réduits.

Le brevet US 4,793,993 décrit un procédé de traitement des fibres kératiniques comprenant la réduction des fibres puis la réticulation des fibres à l'aide de diimidates ou de disuccinimidyl.

Les brevets US 5,300,285 et US 5,270,036 décrivent un procédé comprenant la réduction du cheveu et sa réaction avec une silicone vinyle.

La demande WO 98/38974 décrit un procédé de traitement d'un substrat kératinique comprenant une étape de réduction des liaisons disulfure de la kératine puis la réaction de la kératine avec un composé actif.

Par ailleurs, il est connu de traiter les cheveux par fixation de matière sur cheveux non réduits. Ainsi, le brevet US 5,523,080 décrit le greffage sur les cheveux de polymères portant des fonctions azlactone.

La demande WO-0042010 décrit l'application sur les cheveux d'une composition cosmétique pour le greffage de composés thiolés.

Le brevet US 5,935,560 décrit l'application sur les fibres kératiniques d'une composition permettant le greffage d'une silicone thiolée.

La demande WO 01/00689 décrit le greffage de composés portant des fonctions dihydroxyacétone, N-hydroxy-succinimide, maléimide, phénylazide, benzophénone, amide, esters, diazoesters, diazoamide, imidate, alpha halocétone, pyridyldisulfure, dérivés de carbodimide, vinylsulfone, isocyanate.

La demande WO 00/59458 décrit la réaction sur le cheveu d'un polymère comportant les fonctions succinimide ou glutimide.

Toutefois, ces traitements confèrent des propriétés cosmétiques insuffisamment durables, et notamment peu rémanentes aux shampooings car la matière déposée est rapidement éliminée sous l'action de l'eau et des agents tensioactifs.

La présente invention se propose de remédier aux inconvénients précédemment décrits.

En particulier, la Demanderesse vient de découvrir de façon surprenante qu'un procédé comprenant une étape d'activation non réductrice du cheveu suivie par une étape de fixation sur le cheveu activé d'un composé cosmétiquement actif à l'aide d'une ou plusieurs fonctions du composé permettait de conférer aux cheveux des propriétés cosmétiques durables, et en particulier rémanentes aux shampooings.

Par activation du cheveu, on entend que le cheveu est soumis à un traitement entraînant une modification de la surface du cheveu, de sorte qu'un composé cosmétiquement actif puisse se fixer ensuite sur le cheveu activé à l'aide d'une liaison covalente. L'activation est notamment chimique, ou physique et chimique.

La Demanderesse a en outre constaté que les cheveux ainsi traités présentaient des propriétés cosmétiques durables, comme la brillance, le reflet, la douceur, le lissage, la souplesse ou la coloration, suivant le composé cosmétiquement actif employé.

Elle a également découvert que sur des cheveux ayant auparavant subi des traitements dégradants, comme la décoloration, et présentant une surface abîmée, le procédé selon l'invention conférait au cheveu une protection de sa surface et un toucher agréable.

Elle a constaté d'autres effets cosmétiques liés procédé selon l'invention, comme la rémanence de la couleur, notamment vis-à-vis du décapage entraîné par le lavage, une amélioration de la tenue de la mise en forme, due à une modification de la prise d'eau ou d'humidité, ainsi qu'une protection solaire accrue.

D'autres objets de l'invention apparaîtront à la lecture de la description et des exemples qui suivent.

La présente invention a donc pour objet un procédé de traitement cosmétique capillaire, caractérisé en ce qu'il comprend :
a) une étape non réductrice d'activation des cheveux, étape d'activation chimique ou physique et chimique, par application sur les cheveux de polymères spécifiques selon la revendication 1, et
b) une étape consistant à appliquer sur les cheveux activés au moins un composé cosmétiquement actif susceptible de former une liaison covalente avec les cheveux activés à l'aide d'une ou plusieurs fonctions du composé, de façon à greffer le composé cosmétiquement actif sur les cheveux activés.

La ou les fonctions comportent de préférence au moins un centre électrophile. De cette façon, la création de la liaison covalente s'effectue à partir du ou des centres électrophiles.

La ou les fonctions sont encore plus préférentiellement choisies parmi les fonctions suivantes:
- époxyde,
- aziridine,
- vinyle et vinyles activés, issues des composés acrylonitrile, esters acrylique et méthacrylique, acide et ester crotonique, acide et ester cinnamique, styrène et ses dérivés, butadiène, éthers de vinyle, vinyl cétone, esters maléiques,
- acide carboxylique,
- acétal, hémi-acétal,
- disulfure,
- aminal, hémi-aminal,
- les carbonates cycliques,
- lactone, thiolactone et azlactone,
- thioéther,
- thiocyanate,
- imine,
- succinimide, glutimide,
- oxazine, oxazoline,
- cétone,
- oxazinium, oxazolinium,
- aldéhyde,
- les fonctions de formule -RX, où R désigne un radical alkyle ou aryle ou aralkyle, X désigne I, Br, Cl (fonctions halogénure d'alkyle, d'aryle ou d'aralkyle) ; -OSO₃R' où R' désigne H, un radical alkyle ; -OSO₂R'' où R" désigne H, un radical alkyle ou aryle ; -N⁺(R''')₃ où R"' désigne un radical alkyle ou aryle ;
   -OPO(OR'''')₂ où R'''' désigne H, un radical alkyle,
- les fonctions halogénures de cycle insaturé, le cycle pouvant être un cycle carboné ou un hétérocycle, de formule - RX, R étant un radical cyclique carboné insaturé ou un radical hétérocyclique insaturé et X désignant I, Br, Cl. A titre d'exemple on peut citer la fonction chlorotriazine, chloropyrimidine, chloroquinoxaline, chlorobenzotriazole,
- les fonctions de formule -SO₂X, où X désigne F, Cl (halogénures de sulfonyle); -OSO₃R', où R' désigne H ou un radical alkyle ; -SO₂R'' où R" désigne H, un radical alkyle ou aryle ; -N⁺(R''')₃ où R"' désigne un radical alkyle ou aryle ; -OPO(OR'''')₂ où R"" désigne H, un radical alkyle.

Pour ces fonctions encore plus préférentiellement choisies, la création de la liaison covalente avec les cheveux activés s'effectue à partir du ou des centres électrophiles de la fonction.

L'étape d'activation des cheveux est notamment une étape d'activation chimique, ou physique et chimique.

L'activation physique du cheveu peut consister à soumettre les cheveux à la chaleur, aux ondes électromagnétiques, aux champs électriques, aux ondes acoustiques ou aux plasmas.

L'activation chimique du cheveu peut consister à appliquer sur les cheveux au moins un composé susceptible d'activer les cheveux de manière non réductrice.

La création de la liaison covalente entre le composé cosmétiquement actif et les cheveux activés peut être obtenue à l'issue d'une réaction de substitution nucléophile, électrophile ou radicalaire, d'addition sur des doubles liaisons ou des triples liaisons carbone-carbone ou carbone-hétéroatome, ou d'une réaction d'ouverture de cycle.

La réaction entre le composé cosmétiquement actif et les cheveux activés peut s'effectuer spontanément ou par activation par la température, le pH, un coréactif, ou un catalyseur chimique.

Le composé cosmétiquement actif peut être choisi parmi les molécules simples, les polymères portant une seule fonction susceptible de former une liaison covalente avec les cheveux activés, les particules, les vésicules. Il peut notamment être choisi parmi les dérivés de colorants, de filtres solaires, de vitamines, de peptides, d'osides, d'agents connus pour leurs propriétés hydratantes, comme la glycérine, rafraîchissantes, comme le menthol, ou bénéfiques pour le cheveu comme le panthénol.

A titre de propriétés cosmétiques du cheveu conférés par le composé cosmétiquement actif, on peut citer la douceur et la souplesse conférés par le polydiméthylsiloxane ou le diméthicone polyol, le lissage et la douceur conférés par les polysaccharides tels que les guars, l'anti-salissure conférée par les composés fluorés, les propriétés de résistance à l'humidité conférées par les chaînes alkyle linéaires saturées comportant plus de douze atomes de carbone, les effets de coloration et de reflets conférés par les molécules colorées, les effets de surface (friction, reliefs) ou d'optique conférés par les particules, les effets bénéfiques apportés aux cheveux dégradés par les molécules connues pour leurs propriétés hydratantes ou conditionnantes comme la glycérine, la lanoline ou encore les alcools gras.

Il est également possible d'employer simultanément plusieurs composés cosmétiquement actifs, afin de conférer plusieurs propriétés cosmétiques simultanément.

Lorsque le composé cosmétiquement actif est un polymère, il peut être synthétisé par des réactions radicalaires, de condensation ou d'ouverture de cycle. On entend par polymère un composé comportant au moins cinq motifs de répétition enchaînés par des liaisons covalentes.

Les polymères synthétisés par réaction radicalaire peuvent être choisis parmi les polyacrylates, les polyméthacrylates, les polyvinyles.

Les polymères synthétisés par réaction de condensation peuvent être choisis parmi les polyesters, les polyéthers, les polyamides, les polyuréthanes, le polydiméthylsiloxane, la polypeptide.

Les polymères synthétisés par réaction d'ouverture de cycle peuvent être choisis parmi la polyalkylèneimine, les polyesters.

Les polymères peuvent également être choisis parmi les polymères d'origine naturelle, modifiés chimiquement ou non, comme par exemple les polysaccharides comme la cellulose, le dextrane, le chitosane, le guar et leurs dérivés hydroxyalkylés, carboxyméthylés, aminés, thiolés, ou leurs dérivés à fonction aldéhyde et époxy.

Les polymères peuvent se présenter sous tout type de topologie. Ils peuvent notamment se présenter sous forme linéaire, ramifiée, en étoile ou sous forme hyperbranchée comme les dendrimères.

Lorsque le composé cosmétiquement actif est un polymère, la fonction susceptible de former une liaison covalente avec les cheveux activés du composé cosmétiquement actif peut être présente sur la chaîne polymérique, en bout de chaîne, greffée le long de la chaîne principale ou sur une des chaînes secondaires, sur une branche des polymères en étoile ou hyperbranchés.

Les composés susceptibles d'activer les cheveux de manière non réductrice sont choisis parmi les polymères non réducteurs.

A titre de polymères non réducteurs on peut citer les polyamines, les polysaccharides.

Le composé susceptible d'activer les cheveux de manière non réductrice peut être susceptible de se lier aux cheveux par une liaison hydrogène ou par adsorption.

Le composé susceptible d'activer les cheveux de manière non réductrice peut être choisi parmi tout composé susceptible de s'adsorber sur le cheveu de façon durable et de présenter des fonctions susceptibles de réagir avec les fonctions de greffage du composé cosmétiquement actif. Ces fonctions sont essentiellement des fonctions hydroxyles, amines, ou acides.

Le composé susceptible d'activer les cheveux de manière non réductrice peut ainsi être choisi parmi les polymères filmogènes à fonctions amines qui ont naturellement une bonne affinité pour le cheveu, comme la polyalkylèneimine et ses dérivés, les polyalkylamines, les polylysines, les polyallylamines, les polyglutamines, les polymères hyperbranchés, les dendrimères à terminaisons amines et leurs dérivés.

Les polysaccharides naturels ou modifiés, comme le guar, le dextrane, la cellulose, le chitosane, constituent également un composé susceptible d'activer les cheveux de manière non réductrice intéressant, car ils possèdent déjà des propriétés cosmétiques en dépôt sur le cheveu.

Le composé susceptible d'activer les cheveux de manière non réductrice est de préférence choisi parmi :
- les dendrimères dont les extrémités de chaînes sont des amines primaires, en particulier les polyamidoamines tels que ceux vendus sous la dénomination STARBUST® par la société DENDRITECH,
- les dendrimères ou polymères hyperbranchés à fonction hydroxy tels que ceux vendus sous la dénomination NOVOLAK® par la société CLARIANT,
- la polyéthylèneimine telle que celle vendue sous la dénomination LUPASOL® par la société BASF,
- la polylysine, telle que celle vendue sous la dénomination εPOLYLYSINE® par la société CHISSO,
- la HP cellulose, telle que celle vendue sous la dénomination KLUCEL EF® par la société AQUALON,
- l'amino-dextrane, tel que celui vendu par la société CARBOMER,
- le Chitosane,
- le carboxyméthyl Dextrane, tel que celui vendu par la société FLUKA,
- le carboxyméthyl Chitosane, tel que celui vendu sous la dénomination OLEVASAN® par la société SINO LION.

L'association composé susceptible d'activer les cheveux de manière non réductrice / composé cosmétiquement actif peut notamment être choisie parmi les associations suivantes :
- polyéthylèneimine / dérivé fluorescent comportant un groupement de formule -SO₂Cl ou un groupement chlorotriazine, thiolactone,
- polyéthylèneimine / polydiméthylsiloxane à fonction époxy monofonctionnel,
- Chitosane / sucre sous forme aldéhyde,

Il est également possible d'utiliser plusieurs composés susceptibles d'activer les cheveux de manière non réductrice.

Le procédé de traitement cosmétique capillaire comprend généralement les étapes suivantes :
a) une étape consistant soit à traiter les cheveux à l'aide d'un procédé physique d'activation pendant un temps suffisant pour activer le cheveu, par exemple de 1 à 60 mn, soit à appliquer sur les cheveux pendant un temps de pose de 1 à 60 mn au moins un composé susceptible d'activer les cheveux de manière non réductrice, éventuellement sous l'action de la chaleur,
b) puis éventuellement après rinçage une étape consistant à appliquer sur les cheveux ainsi activés pendant un temps de pose de 1 à 60 mn au moins un composé cosmétiquement actif susceptible de former une liaison covalente avec les cheveux activés.

Les exemple suivants sont destinés à illustrer l'invention.

### Exemple 1 : greffage d'un composé cosmétiquement actif sur un enduit de polyéthylèneimine (PEI)

### Solutions employées :

| **Solution A** | |
|---|---|
| Polyéthylèneimine (PM25000) vendue sous la dénomination Lupasol® par la société BASF | 10 g |
| HCl 36% | qsp pH 8 |
| H₂O | qsp 100g |

| **Solution B** | |
|---|---|
| Colorant Reactive Blue 4 | 5g |
| H₂O | qsp 100g |

Le colorant Reactive Blue 4 est un colorant à fonction chlorotriazine, de formule suivante :

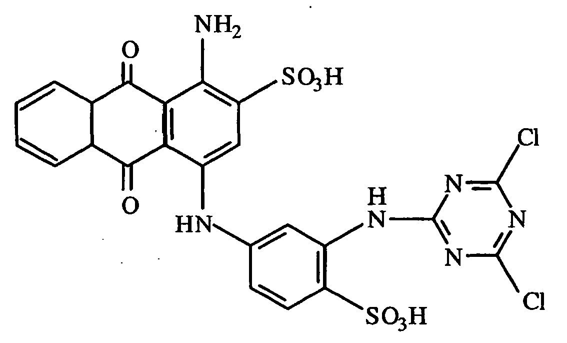

On applique 0,4 g de solution A sur les mèches (2,5g). Les mèches sont portées à 60°C pendant 30 mn. Sans rinçage intermédiaire, on applique 0,4 g de solution B sur les mèches. Les mèches sont alors portées à 30°C pendant 30 mn.

Les mèches sont ensuite rincées à l'eau et séchées. On obtient des mèches colorées avec une intensité de coloration supérieure à celle obtenue avec des mèches traitées uniquement avec la solution B. Cette couleur est en outre rémanente aux shampooings.

### Exemple 2 : greffage de glucose sur un enduit de polyéthylèneimine (PEI)

### Solutions employées :

| **Solution A** | |
|---|---|
| Polyéthylèneimine (PM750000) vendue sous la dénomination Lupasol® par la société BASF | 5 g |
| H₂O | qsp 100g |

| **Solution B** | |
|---|---|
| Glucose | 5g |
| H₂O | qsp 100g |

On applique 0,4 g de solution A sur les mèches (2,5g). Les mèches sont portées à 60°C pendant 30 mn. Sans rinçage intermédiaire, on applique 20 ml de solution B sur les mèches. On rajoute ensuite 0,12g de NaBH₃CN. Les mèches sont alors portées à température ambiante pendant 16h.

Les mèches sont ensuite lavées au shampooing, rincées à l'eau et séchées. On obtient des mèches colorées présentant beaucoup de masse et de corps à l'état humide et une douceur marquée à l'état sec. Ces effets sont en outre rémanents aux shampooings.

## Revendications

1. Procédé de traitement cosmétique capillaire **caractérisé en ce qu'**il comprend :
a) une étape non réductrice d'activation des cheveux, qui est une étape d'activation, chimique ou physique et chimique, l'activation chimique consistant à appliquer sur les cheveux au moins un composé susceptible d'activer les cheveux de manière non réductrice et choisi parmi la polyalkylèneimine et ses dérivés, les polyalkylamines, les polylysines, les polyallylamines, les polyglutamines, les polymères hyperbranchés, les dendrimères à terminaisons amines et leurs dérivés, les polysaccharides naturels ou modifiés, et
b) une étape consistant à appliquer sur les cheveux activés au moins un composé cosmétiquement actif susceptible de former au moins une liaison covalente avec les cheveux activés à l'aide d'une ou plusieurs fonctions du composé.

2. Procédé selon la revendication 1, **caractérisé en ce que** la ou les fonctions du composé cosmétiquement actif comportent au moins un centre électrophile.

3. Procédé selon la revendication 1 ou 2, **caractérisé en ce que** la ou les fonctions du composé cosmétiquement actif sont choisies parmi les fonctions suivantes :
- époxyde,
- aziridine,
- vinyle et vinyles activés, issues des composés acrylonitrile, esters acrylique et méthacrylique, acide et ester crotonique, acide et ester cinnamique, styrène et ses dérivés, butadiène, éthers de vinyle, vinyl cétone, esters maléiques,
- acide carboxyliqué,
- acétal, hémi-acétal,
- disulfure,
- aminal, hémi-aminal,
- les carbonates cycliques,
- lactone, thiolactone et azlactone,
- thioéther,
- thiocyanate,
- imine,
- succinimide, glutimide,
- oxazine, oxazoline,
- cétone,
- oxazinium, oxazolinium,
- aldéhyde,
- les fonctions de formule -RX, où R désigne un radical alkyle ou aryle ou aralkyle, X désigne I, Br, Cl ; -OSO₃R' où R' désigne H, un radical alkyle ; -OSO₂R'' où R" désigne H, un radical alkyle ou aryle ; -N⁺(R''')₃ où R''' désigne un radical alkyle ou aryle ;
-OPO(OR'''')₂ où R"" désigne H, un radical alkyl,
- les fonctions halogénures de cycle insaturé, le cycle pouvant être un cycle carboné ou un hétérocycle, de formule -RX, R étant un radical cyclique carboné insaturé ou un radical hétérocyclique insaturé et X désignant I, Br, Cl,
- les fonctions de formule -SO₂X, où X désigne F, Cl ; -OSO₃R', où R' désigne H ou un radical alkyle ; -SO₂R'' où R'' désigne H, un radical alkyle ou aryle ; -N⁺(R''')₃ où R''' désigne un radical alkyle ou aryle ; -OPO(OR'''')₂ où R"" désigne H, un radical alkyle.

4. Procédé selon la revendication 1, **caractérisée en ce que** l'étape d'activation physique consiste à soumettre les cheveux à la chaleur, aux ondes électromagnétiques, aux champs électriques, aux ondes acoustiques ou aux plasmas.

5. Procédé selon la revendication 1, **caractérisé en ce que** l'étape d'activation des cheveux est une étape d'activation chimique.

6. Procédé selon l'une quelconque des revendications précédentes, **caractérisé en ce que** la création de la liaison covalente entre le composé cosmétiquement actif et les cheveux activés est obtenue à l'issue d'une réaction de substitution nucléophile, électrophile ou radicalaire, d'addition sur des doubles liaisons ou des triples liaisons carbone-carbone ou carbone-hétéroatome, ou d'une réaction d'ouverture de cycle.

7. Procédé selon l'une quelconque des revendications précédentes, **caractérisé en ce que** la réaction entre le composé cosmétiquement actif et les cheveux activés s'effectue spontanément ou par activation par la température, le pH, un coréactif, ou un catalyseur chimique.

8. Procédé selon l'une quelconque des revendications précédentes, **caractérisé en ce que** le composé cosmétiquement actif est choisi parmi les molécules simples, les polymères portant une seule fonction susceptible de former une liaison covalente avec les cheveux activés, les particules, les vésicules.

9. Procédé selon la revendication 8, **caractérisé en ce que** le composé cosmétiquement actif est choisi parmi les dérivés de colorants, de filtres solaires, de vitamines, de peptides, d'osides, d'agents connus pour leurs propriétés hydratantes, rafraîchissantes, ou bénéfiques pour le cheveu.

10. Procédé selon la revendication 8, **caractérisé en ce que** le composé cosmétiquement actif est un polymère synthétisé par des réactions radicalaires, de condensation ou d'ouverture de cycle.

11. Procédé selon la revendication 10, **caractérisé en ce que** les polymères synthétisés par des réactions radicalaires sont choisis parmi les polyacrylates, les polyméthacrylates, les polyvinyles.

12. Procédé selon la revendication 10, **caractérisé en ce que** les polymères synthétisés par réaction de condensation sont choisis parmi les polyesters, les polyéthers, les polyamides, les polyuréthanes, le polydiméthylsiloxane, la polypeptide.

13. Procédé selon la revendication 10, **caractérisé en ce que** les polymères synthétisés par réaction d'ouverture de cycle sont choisis parmi la polyalkylèneimine et les polyesters.

14. Procédé selon la revendication 8, **caractérisé en ce que** les polymères sont choisis parmi les polymères d'origine naturelle, modifiés chimiquement ou non.

15. Procédé selon la revendication 14, **caractérisé en ce que** les polymères sont choisis parmi la cellulose, le dextrane, le chitosane, le guar et leurs dérivés hydroxyalkylés, carboxyméthylés, aminés, thiolés, ou leurs dérivés à fonction aldéhyde et époxy.

16. Procédé selon la revendication 8, **caractérisé en ce que** les polymères se présentent sous forme linéaire, ramifiée, en étoile ou sous forme hyperbranchée.

17. Procédé selon la revendication 1, **caractérisé en ce que** le composé susceptible d'activer les cheveux de manière non réductrice est susceptible de se lier aux cheveux par une liaison hydrogène ou par adsorption.

18. Procédé selon la revendication 1, **caractérisé en ce que** le composé susceptible d'activer les cheveux de manière non réductrice est choisi parmi :
- les dendrimères dont les extrémités de chaîne sont des amines primaires,
- les dendrimères ou polymères hyperbranchés à fonction hydroxy,
- la polyéthylèneimine,
- la polylysine,
- la HP cellulose,
- l'amino-dextrane,
- l'alcool polyvinylique,
- l'alcool amino polyvinylique,
- le Chitosane,
- le carboxyméthyl Dextrane,
- le carboxyméthyl Chitosane.

19. Procédé selon l'une quelconque des revendications précédentes, **caractérisé en ce qu'**il comprend les étapes suivantes :
a) une étape consistant soit à appliquer sur les cheveux pendant un temps de pose de 1 à 60 mn au moins un composé susceptible d'activer les cheveux de manière non réductrice, éventuellement sous l'action de la chaleur,
b) puis éventuellement après rinçage une étape consistant à appliquer sur les cheveux ainsi activés pendant un temps de pose de 1 à 60 mn au moins un composé cosmétiquement actif susceptible de former une liaison covalente avec les cheveux activés.

## Claims

1. Cosmetic hair treatment process, **characterized in that** it comprises:
a) a non-reducing step of activation of the hair, which is a step of chemical or physical and chemical activation, the chemical activation consisting in applying to the hair at least one compound capable of non-reducingly activating the hair and chosen from polyalkyleneimine and its derivatives, polyalkylamines, polylysines, polyallylamines, polyglutamines, hyperbranched polymers, dendrimers containing amine end groups and derivatives thereof, natural or modified polysaccharides, and
b) a step that consists in applying to the activated hair at least one cosmetically active compound capable of forming at least one covalent bond with the activated hair with the aid of one or more functions of the compound.

2. Process according to Claim 1, **characterized in that** the function(s) of the cosmetically active compound comprise(s) at least one electrophilic centre.

3. Process according to Claim 1 or 2, **characterized in that** the function(s) of the cosmetically active compound is(are) chosen from the following functions:
- epoxide,
- aziridine,
- vinyl and activated vinyls, derived from acrylonitrile compounds, acrylic and methacrylic esters, crotonic acid and ester, cinnamic acid and ester, styrene and its derivatives, butadiene, vinyl ethers, vinyl ketone and maleic esters,
- carboxylic acid,
- acetal or hemiacetal,
- disulfide,
- aminal or hemiaminal,
- cyclic carbonates,
- lactone, thiolactone and azlactone,
- thioether,
- thiocyanate
- imine,
- succinimide or glutimide,
- oxazine or oxazoline,
- ketone,
- oxazinium or oxazolinium,
- aldehyde,
- functions of formula -RX, in which R denotes an alkyl or aryl or aralkyl radical, X denotes I, Br or Cl; -OSO₃R' in which R' denotes H, an alkyl radical; -OSO₂R" in which R" denotes H or an alkyl or aryl radical; -N⁺(R''')₃ in which R''' denotes an alkyl or aryl radical;
-OPO(OR'''')₂ in which R'''' denotes H or an alkyl radical,
- the halide functions of an unsaturated ring, the ring possibly being a carbon-based ring or a heterocycle, of formula -RX, R being an unsaturated carbocyclic radical or an unsaturated heterocyclic radical and X denoting I, Br or Cl,
- functions of formula -SO₂X, in which X denotes F or Cl; -OSO₃R', in which R' denotes H or an alkyl radical; -SO₂R'' in which R'' denotes H or an alkyl or aryl radical; -N'(R''')₃ in which R''' denotes an alkyl or aryl radical;
-OPO(OR'''')₂ in which R'''' denotes H or an alkyl radical.

4. Process according to Claim 1, **characterized in that** the physical activation step consists in subjecting the hair to heat, electromagnetic waves, electric fields, acoustic waves or plasmas.

5. Process according to Claim 1, **characterized in that** the activation step of the hair is a step of chemical activation.

6. Process according to any one of the preceding claims, **characterized in that** the creation of the covalent bond between the cosmetically active compound and the activated hair is obtained after a nucleophilic, electrophilic or free-radical substitution reaction, an addition reaction to carbon-carbon or carbon-hetero atom double bonds or triple bonds, or a ring-opening reaction.

7. Process according to any one of the preceding claims, **characterized in that** the reaction between the cosmetically active compound and the activated hair takes place spontaneously or by activation by temperature, by pH, with a coreagent or with a chemical catalyst.

8. Process according to any one of the preceding claims, **characterized in that** the cosmetically active compound is chosen from simple molecules, polymers bearing only one function capable of forming a covalent bond with the activated hair, particles and vesicles.

9. Process according to Claim 8, **characterized in that** the cosmetically active compound is chosen from dye derivatives, sunscreens, vitamins, peptides, saccharide, and agents that are known for their moisturizing, refreshing, or beneficial properties on the hair.

10. Process according to Claim 8, **characterized in that** the cosmetically active compound is a polymer synthesized via free-radical reactions, condensation reactions or ring-opening reactions.

11. Process according to Claim 10, **characterized in that** the polymers synthesized via free-radical reactions are chosen from polyacrylates, polymethacrylates and polyvinyls.

12. Process according to Claim 10, **characterized in that** the polymers synthesized via a condensation reaction are chosen from polyesters, polyethers, polyamides, polyurethanes, polydimethylsiloxane and polypeptide.

13. Process according to Claim 10, **characterized in that** the polymers synthesized via ring-opening reaction are chosen from polyalkyleneimine and polyesters.

14. Process according to Claim 8, **characterized in that** the polymers are chosen from polymers of natural origin, which may or may not have been chemically modified.

15. Process according to Claim 14, **characterized in that** the polymers are chosen from cellulose, dextran, chitosan and guar and the hydroxyalkyl, carboxymethyl, amino and thiol derivatives thereof, or the derivatives thereof containing aldehyde and epoxy functions.

16. Process according to Claim 8, **characterized in that** the polymers are in linear, branched or starburst form or in hyperbranched form.

17. Process according to Claim 1, **characterized in that** the compound capable of non-reducingly activating the hair is capable of binding to the hair by hydrogen bonding or by adsorption.

18. Process according to Claim 1, **characterized in that** the compound capable of non-reducingly activating the hair is chosen from:
- dendrimers whose chain ends are primary amines,
- dendrimers or hyperbranched polymers containing hydroxyl functions,
- polyethyleneimine,
- polylysine,
- HP cellulose,
- aminodextran,
- polyvinyl alcohol,
- polyvinyl amino alcohol,
- chitosan,
- carboxymethyldextran,
- carboxymethylchitosan.

19. Process according to any one of the preceding claims, **characterized in that** it comprises the following steps:
a) a step consisting in applying to the hair for a leave-in time of from 1 to 60 minutes at least one compound capable of non-reducingly activating the hair, optionally under the action of heat,
b) and then optionally after rinsing, a step consisting in applying to the hair thus activated, for a leave-in time of from 1 to 60 minutes, at least one cosmetically active compound capable of forming a covalent bond with the activated hair.

## Patentansprüche

1. Verfahren zur kosmetischen Behandlung der Haare, **dadurch gekennzeichnet, dass** es umfasst:
a) einen nicht reduzierenden Schritt der Aktivierung der Haare, bei dem es sich um einen chemischen oder einen physikalischen und chemischen Schritt der Aktivierung handelt, wobei die chemische Aktivierung darin besteht, auf die Haare mindestens eine Verbindung aufzubringen, die befähigt ist, die Haare auf eine nichtreduzierende Weise zu aktivieren, und die unter Polyalkylenimin und seinen Derivaten, Polyalkylaminen, Polylysinen, Polyallylaminen, Polyglutaminen, hochverzweigten Polymeren, Dendrimeren mit endständigen Aminogruppen und deren Derivaten, natürlichen oder modifizierten Polysacchariden ausgewählt ist, und
b) einen Schritt, der darin besteht, auf die aktivierten Haare mindestens eine kosmetisch wirksame Verbindung aufzubringen, die befähigt ist, mittels einer oder mehrerer Funktionen der Verbindung mindestens eine kovalente Bindung mit den aktivierten Haaren auszubilden.

2. Verfahren nach Anspruch 1, **dadurch gekennzeichnet, dass** die Funktion(en) der kosmetisch wirksamen Verbindung mindestens ein elektrophiles Zentrum besitzen.

3. Verfahren nach Anspruch 1 oder 2, **dadurch gekennzeichnet, dass** die Funktion(en) der kosmetisch wirksamen Verbindung unter den folgenden Funktionen ausgewählt sind:
- Epoxid,
- Aziridin,
- Vinyl oder aktivierten Vinylen, die von Acrylnitrilen, Acrylsäureestern und Methacrylsäureestern, Crotonsäure und Crotonsäureestern, Zimtsäure und Zimtsäureestern, Styrol und seinen Derivaten, Butadien, Vinylethern, Vinylketon, Maleinsäureestern abgeleitet sind,
- Carbonsäure,
- Acetal, Halbacetal,
- Disulfid,
- Aminal, Halbaminal,
- cyclischen Carbonaten,
- Lacton, Thiolacton und Azlacton,
- Thioether,
- Thiocyanat,
- Imin,
- Succinimid, Glutimid,
- Oxazin, Oxazolin,
- Keton,
- Oxazinium, Oxazolinium,
- Aldehyd,
- Funktionen der Formel -RX, worin bedeuten: R eine Alkylgruppe oder Arylgruppe oder Aralkylgruppe und X I, Br, Cl;
- OSO₃R', wobei R' H oder eine Alkylgruppe bedeutet;
- OSO₂R", wobei R" H, eine Alkylgruppe oder eine Arylgruppe bedeutet; -N⁺(R''')₃, wobei R''' eine Alkylgruppe oder eine Arylgruppe bedeutet; -OPO(OR'''')₂, wobei R'''' H oder eine Alkylgruppe bedeutet,
- Halogenidfunktionen mit ungesättigtem Ring der Formel -RX, wobei es sich um einen Kohlenstoffring oder einen Heterocyclus handeln kann und wobei R eine ungesättigte cyclische Gruppe auf Kohlenstoffbasis oder eine ungesättigte heterocyclische Gruppe ist und X I, Br, Cl bedeutet,
- Funktionen der Formel -SO₂X, worin bedeuten: X F, Cl; -OSO₃R', wobei R' H oder eine Alkylgruppe bedeutet; -SO₂R", wobei R" H, eine Alkylgruppe oder eine Arylgruppe bedeutet; -N⁺(R''')₃, wobei R''' eine Alkylgruppe oder eine Arylgruppe bedeutet; -OPO(OR'''')₂, wobei R'''' H oder eine Alkylgruppe bedeutet.

4. Verfahren nach Anspruch 1, **dadurch gekennzeichnet, dass** der Schritt der physikalischen Aktivierung darin besteht, die Haare Wärme, elektromagnetischer Strahlung, elektrischen Feldern, akustischen Wellen oder Plasma auszusetzen.

5. Verfahren nach Anspruch 1, **dadurch gekennzeichnet, dass** der Schritt der Aktivierung der Haare in einer chemischen Aktivierung besteht.

6. Verfahren nach einem der vorhergehenden Ansprüche, **dadurch gekennzeichnet, dass** die Ausbildung der kovalenten Bindung zwischen der kosmetisch wirksamen Verbindung und den aktivierten Haaren nach einer nucleophilen, elektrophilen oder radikalischen Substitution, einer Addition an Kohlenstoff-Kohlenstoff-Doppelbindungen, Kohlenstoff-Kohlenstoff-Dreifachbindungen, Kohlenstoff-Heteroatom-Doppelbindungen oder Kohlenstoff-Heteroatom-Dreifachbindungen oder einer Ringöffnungsreaktion erhalten wird.

7. Verfahren nach einem der vorhergehenden Ansprüche, **dadurch gekennzeichnet, dass** die Reaktion zwischen der kosmetisch wirksamen Verbindung und den aktivierten Haaren spontan erfolgt oder nach Aktivierung durch Temperatur, pH-Wert, einem Coreagenz oder einem chemischen Katalysator.

8. Verfahren nach einem der vorhergehenden Ansprüche, **dadurch gekennzeichnet, dass** die kosmetisch wirksame Verbindung unter den einfachen Molekülen, Polymeren, die eine einzige Funktion aufweisen, die mit den aktivierten Haaren eine kovalente Bindung ausbilden kann, Partikeln und Vesikeln ausgewählt ist.

9. Verfahren nach Anspruch 8, **dadurch gekennzeichnet, dass** die kosmetisch wirksame Verbindung unter den Farbstoffderivaten, Sonnenschutzfiltern, Vitaminen, Peptiden, Zuckern, Stoffen, die für ihre hydratisierenden Eigenschaften bekannt sind, erfrischenden Stoffen oder für die Haare günstigen Stoffen ausgewählt sind.

10. Verfahren nach Anspruch 8, **dadurch gekennzeichnet, dass** die kosmetisch wirksame Verbindung ein Polymer ist, das durch radikalische Reaktionen, Kondensation oder Ringöffnung hergestellt ist.

11. Verfahren nach Anspruch 10, **dadurch gekennzeichnet, dass** die durch radikalische Reaktionen synthetisierten Polymere unter den Polyacrylaten, Polymethacrylaten und Polyvinylen ausgewählt sind.

12. Verfahren nach Anspruch 10, **dadurch gekennzeichnet, dass** die durch Kondensation synthetisierten Polymere unter den Polyestern, Polyethern, Polyamiden, Polyurethanen, Polydimethylsiloxan und Polypeptid ausgewählt sind.

13. Verfahren nach Anspruch 10, **dadurch gekennzeichnet, dass** die durch Ringöffnung synthetisierten Polymere unter Polyalkylenimin und Polyestern ausgewählt sind.

14. Verfahren nach Anspruch 8, **dadurch gekennzeichnet, dass** die Polymere unter den Polymeren natürlicher Herkunft ausgewählt sind, die gegebenenfalls chemisch modifiziert wurden.

15. Verfahren nach Anspruch 14, **dadurch gekennzeichnet, dass** die Polymere unter Cellulose, Dextran, Chitosan, Guargummi und ihren Hydroxyalkylderivaten, Carboxymethylderivaten, Aminoderivaten, Thiolderivaten oder ihren Derivaten mit Aldehydfunktion und Epoxyfunktion ausgewählt sind.

16. Verfahren nach Anspruch 8, **dadurch gekennzeichnet, dass** die Polymere linear, verzweigt, sternförmig verzweigt oder hochverzweigt vorliegen.

17. Verfahren nach Anspruch 1, **dadurch gekennzeichnet, dass** die Verbindung, die befähigt ist, die Haare auf eine nichtreduzierende Weise zu aktivieren, befähigt ist, sich mit den Haaren über eine Wasserstoffbindung oder durch Adsorption zu verbinden.

18. Verfahren nach Anspruch 1, **dadurch gekennzeichnet, dass** die Verbindung, die befähigt ist, die Haare auf eine nichtreduzierende Weise zu aktivieren, ausgewählt ist unter:
- Dendrimeren, deren Kettenenden primäre Amine sind,
- Dendrimeren oder hochverzweigten Polymeren mit Hydroxyfunktion,
- Polyethylenimin,
- Polylysin,
- HP Cellulose,
- Aminodextran,
- Polyvinylalkohol,
- Aminopolyvinylalkohol,
- Chitosan,
- Carboxymethyldextran,
- Carboxymethylchitosan.

19. Verfahren nach einem der vorhergehenden Ansprüche, **dadurch gekennzeichnet, dass** es die folgenden Schritte umfasst:
a) einen Schritt, der darin besteht, mindestens eine Verbindung, die befähigt ist, die Haare auf eine nichtreduzierende Weise zu aktivieren, gegebenenfalls unter Einwirkung von Wärme während einer Einwirkzeit von 1 bis 60 min auf die Haare aufzubringen,
b) und dann gegebenenfalls nach einem Spülschritt einen Schritt, der darin besteht, auf die auf diese Weise aktivierten Haare mindestens eine kosmetisch wirksame Verbindung, die befähigt ist, eine kovalente Bindung mit den aktivierten Haaren auszubilden, während einer Einwirkzeit von 1 bis 60 min aufzubringen.
